# EUROPÄISCHE PATENTANMELDUNG

(11) **EP 4 046 663 A2**
(43) Veröffentlichungstag der Anmeldung: **24.08.2022**
(21) Anmeldenummer: 22154518.9
(22) Anmeldetag: 01.02.2022
(51) Int. Cl.: A61L 9/20, F24F 8/22

(54) **MODULARES SYSTEM ZUR RAUMLUFTDESINFEKTION BZW. ENTKEIMUNG VON LUFT DURCH BEHANDLUNG MIT ULTRAVIOLETTER STRAHLUNG**

(30) Priorität: 04.12.2020 DE 202020106999 U
(71) Anmelder: biotec Umwelt-Analytik-Beratung-Service GmbH, 33332 Gütersloh (DE); Bermpohl, Andreas, 33332 Gütersloh (DE); Weisser, Jörg, 33332 Gütersloh (DE); Weisser, Frank, 33332 Gütersloh (DE)
(72) Erfinder: Bermpohl, Dr., Andreas, 33332 Gütersloh (DE); Weißer, Jörg, 33332 Gütersloh (DE); Weißer, Frank, 33332 Gütersloh (DE)
(74) Vertreter: Grund, Martin

(57) **Zusammenfassung**

Die Erfindung betrifft ein modulares System, umfassend mindestens eine Vorrichtung (UV-C-Modul) zur Raumluftdesinfektion bzw. Entkeimung von Luft durch Behandlung mit ultravioletter (UV-C-) Strahlung.

## Beschreibung

### Gebiet der Erfindung

Die Erfindung betrifft ein modulares System, umfassend mindestens eine Vorrichtung (UV-C-Modul) zur Raumluftdesinfektion bzw. Entkeimung von Luft durch Behandlung mit ultravioletter (UV-C-) Strahlung.

### Hintergrund der Erfindung

Zur Entkeimung von Luft in gewerblich und industriell genutzten Räumen, sowie in öffentlichen Räumen, wie etwa Krankenhäuser und Schulen, werden häufig Geräte verwendet, die einen UV-C-Strahler umfassen.

Solche Luftreinigungsgeräte umfassen einen linearen Strömungskanal, durch den die Luft geleitet wird, und mindestens einen UV-C-Strahler, der im Strömungskanal angeordnet ist. Die vorbeiströmende Luft wird somit UV-C-Strahlung ausgesetzt, die in der Luft enthaltene Krankheitserreger abtötet bzw. inaktiviert. Solche Luftreinigungsgeräte sind z.B. in DE-U 93 13 409.6, DE-PS 26 18 127 und DE-PS 32 08 519 beschrieben.

Meist sind solche Luftreinigungsgeräte jedoch fest im Raum verbaut und erlauben keinen flexiblen oder kurzfristigen Einsatz.

Die vorliegende Erfindung löst nun dieses Problem, indem sie durch ein modulares autonomes System eine Lösung für den kurzfristigen, flexiblen und bedarfsgerechten Einsatz solcher Luftreinigungsgeräte bereitstellt.

### Zusammenfassung der Erfindung

Die vorliegende Erfindung stellt ein modulares System zur effektiven Desinfektion bzw. Entkeimung von Raumluft mittels UV-C-Strahlung bereit, umfassend mindestens ein UV-C-Modul.

Die vorliegende Erfindung stellt zunächst ein UV-C-Modul zur Desinfektion bzw. Entkeimung von Raumluft mittels UV-C-Strahlung bereit, umfassend mindestens einen Strömungskanal, eine erste Lichtfalle, einen Ventilator, ein Filterflies, mindestens einen UV-C-Strahler und eine zweite Lichtfalle,
wobei die den Strömungskanal durchströmende Luft mit UV-C-Strahlung behandelt und die darin enthaltenen Keime inaktiviert werden.

In einer bevorzugten Ausführungsform der Erfindung ist das UV-C- Modul rechteckig oder quadratisch ausgestaltet.

In einer anderen bevorzugten Ausführungsform umfasst das UV-C-Modul einen geräuschoptimierten Ventilator.

In einer weiteren bevorzugten Ausführungsform umfasst der Ventilator eine Drehzahlregelung und wird bei nur 20 - 35 %, vorzugsweise 20 % der maximalen Ventilatorleistung betrieben. Dadurch kann eine deutliche Geräuschreduzierung erzielt werden.

Die Erfindung stellt ferner ein modulares System für die Desinfektion bzw. Entkeimung von Raumluft durch Behandlung mit ultravioletter (UV) Strahlung bereit, umfassend mindestens ein UV-C-Modul wie hierin offenbart.

In einer bevorzugten Ausführungsform umfasst das System zwischen 2 und 8 UV-C-Module.

In einer weiteren bevorzugten Ausführungsform umfasst das System 2, 3 oder 4 UV-C-Module. Bevorzugte Ausführungsformen mit 2, 3 oder 4 UV-C- Modulen sind in den Abbildungen exemplarisch veranschaulicht.

In einer weiteren bevorzugten Ausführungsform umfasst das System 6 UV-C-Module.

In einer anderen Ausführungsform umfasst das System ferner einen Standfuß und ist als Standgerät ausgestaltet.

In einer anderen Ausführungsform umfasst das System ferner eine Wandaufhängung und ist zur Wandmontage ausgestaltet oder umfasst eine Deckenaufhängung und ist zur Deckenaufhängung ausgestaltet.

Die Erfindung umfasst weiterhin kombiniertes Desinfektionssystem (nachfolgend auch als Desinfektionsinsel bezeichnet) bestehend aus einem vorstehend beschriebenen modularen System umfassend vorzugsweise 1, 2, 3 oder 4 oder mehr UV-C-Module und weiter umfassend einen Standfuß oder Ständer, vorzugsweise aus Edelstahl und ein Desinfektionsmodul zur Hautdesinfektion, welches ebenfalls am dem Standfuß oder Ständer befestigt wird. Vorzugsweise werden die UV-C-Module und das Desinfektionsmodul zur Hautdesinfektion auf gegenüberliegenden Seiten des Standfußes bzw. Ständers montiert.

In einer bevorzugten Ausführungsform umfasst das System ferner ein Schallschutzgehäuse oder einen elektrischen Schallwandler.

Die Erfindung stellt ferner ein Montageblech für den Aufbau eines UV-C-Moduls bereit, umfassend Steckplätze für eines oder mehrere von mindestens ein Gitter, eine Lichtfalle, einen Ventilator, ein Filterflies und einen UV-C-Strahler.

In einer bevorzugten Ausführungsform des Montageblechs sind die Steckplätze in der folgenden Reihenfolge angeordnet:
1) Gitter
2) Lichtfalle
3) Ventilator
4) Filterflies
5) UV-C-Strahler
6) Lichtfalle
7) Gitter.

In einer anderen bevorzugten Ausführungsform des Montageblechs sind die Steckplätze in der folgenden Reihenfolge angeordnet:
1) Gitter
2) Lichtfalle
3) Filterflies
4) Ventilator
5) UV-C-Strahler
6) Lichtfalle
7) Gitter.

Weiterhin stell die Erfindung einen Bausatz für ein UV-C-Modul bereit, umfassend das Montageblech wie hierin beschrieben und mindestens ein Gitter, eine Lichtfalle, einen Ventilator, ein Filterflies und einen UV-C-Strahler.

In einer bevorzugten Ausführungsform umfasst der Bausatz ferner ein Schallfiltergehäuse oder einen elektrischen Schallwandler, der mit dem UV-C-Modul verbunden werden kann.

In einer bevorzugten Ausführungsform umfasst der Bausatz ferner einen geräuschoptimierten Ventilator.

In einem weiteren bevorzugten Ausführungsform umfasst der Bausatz ferner mindestens eines der folgenden: eine Wandhalterung, einen Standfuß oder eine Deckenaufhängung.

### Kurze Beschreibung der Zeichnungen

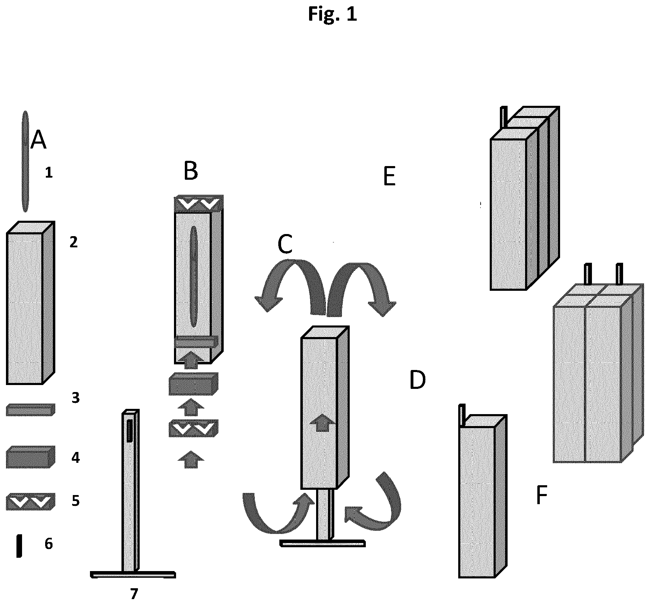

Fig. 1 zeigt schematisch eine Übersicht über verschiedene Ausführungsformen eines oder mehrere UV-C-Module eines modularen Systems der Erfindung. A) Dargestellt sind ein einzelnes UV-C-Modul und seine Bestandteile (in Einzelteilen). B) Dargestellt ist ein einzelnes UV-C-Modul (zusammengebaut). C) Dargestellt ist ein UV-C-Modul, das als Ein-Modul-Standgerät ausgearbeitet ist. D) Dargestellt ist ein UV-C-Modul, das als Ein-Modul-Wandgerät ausgearbeitet ist. E) Dargestellt ist ein UV-C-Gerät, das als Drei-Modul-Wandgerät ausgearbeitet ist. F) Dargestellt ist ein UV-C-Gerät, das als Vier-Modul-Wandgerät ausgearbeitet ist.
Fig. 2 zeigt schematisch eine weitere Ausführungsform des modularen Systems der Erfindung. Dargestellt ist ein Kombinationsgerät umfassend ein Ein-Modul-Standgerät, sowie ein Gerät zur Hautdesinfektion (Desinfektionsinsel).
Fig. 3 zeigt schematisch weitere Ausführungsformen des modularen Systems der Erfindung. A) Dargestellt ist ein Vier-Modul-Gerät, das zusätzlich ein Außengehäuse zur Schallisolierung umfasst. B) Dargestellt ist ein Ein-Modul-Gerät, das als Ein-Modul-Hängegerät ausgearbeitet ist.
Fig. 4 zeigt schematisch die Integration sechs einzelner UV-C-Module in einer zentralen Raumlufttechnik (RLT) -Anlage.
Fig. 5 zeigt schematisch ein Montageblech, auf dem die einzelnen Komponenten eines UV-C-Moduls aufgebracht werden.

### Ausführliche Beschreibung

### Definitionen

*Desinfizierung bzw. Entkeimung:* Der Begriff Desinfektion bzw. Entkeimung wie hierin verwendet bezieht sich auf die Inaktivierung bzw. Abtötung von Krankheitserregern im Medium Luft um mindestens 99%. Die Begriffe Desinfektion und Entkeimung werden hierhin äquivalent verwendet.

*Krankheitserreger und Keime:* Krankheitserreger wie hierin verwendet bezieht sich auf Mikroorganismen und subzelluläre Erreger, die Krankheiten verursachen können. Hierhin bezieht sich der Begriff vor allem auf Mikroorganismen und subzelluläre Erreger, die Krankheiten in Säugetieren, insbesondere dem Menschen verursachen können. Hierzu gehören vor allem Algen, Bakterien, Parasiten, Pilze, Prionen, Protozoen, Viren und Viroide.

*UV oder UV-Strahlung:* Die Begriffe UV, UV-Strahlung, UV-C oder UV-C-Strahlung werden hierin äquivalent verwendet. Sie beschreiben elektromagnetische Strahlung im kurzen Wellenlängenbereich. Hierhin wird der Begriff für UV-C-Strahlung im Bereich von um 260 nm verwendet, bevorzugt um 253,7 nm.

*UV-C-Modul und Vorrichtung:* Der Begriff UV-C-Modul wie hierin verwendet beschreibt eine Vorrichtung zur Desinfektion bzw. Entkeimung von Raumluft. Die Begriffe UV-C-Modul und Vorrichtung werden somit äquivalent verwendet.

*UV-C-Strahler und UV-C-Lampen:* Der Begriff UV-C-Strahler wie hierin verwendet beschreibt die UV-C-Quelle, die in einem UV-C-Modul der Erfindung eingesetzt wird um desinfizierende bzw. entkeimende Strahlung zu erzeugen. Die Begriffe UV-C-Strahler und UV-C-Lampe werden äquivalent verwendet.

### Detaillierte Beschreibung

Die vorliegende Erfindung stellt ein modulares System bereit, das mindestens eine Vorrichtung (UV-C-Modul) umfasst, die mittels UV-Strahlung die effektive Desinfektion bzw. Entkeimung von Raumluft gewährleistet. Ferner werden ein Bausatz, sowie ein Montageblech für den einfachen Aufbau eines UV-C-Moduls bereitgestellt.

Der Wirkungsmechanismus der Desinfektion durch UV-Strahlung beruht dabei auf der photochemischen Veränderung von Teilen der DNA bei Bestrahlung mit Wellenlängen im UV-C-Bereich um 260 Nanometer, insbesondere bei 253,7 nm. Die photochemische Veränderung ist die Dimerisierung benachbarter Thyminbasen an der DNA. Zellen bzw. Mikroorganismen verlieren damit sofort ihre Vermehrungsfähigkeit, es kommt zur Keimreduzierung.

In der vorliegenden Erfindung wird die UV-Strahlung, die auf die Raumluft einwirken soll, nun durch eine Vorrichtung bereitgestellt. Diese Vorrichtung ist auch als UV-C-Modul bezeichnet. Das UV-C-Modul ist vielseitig einsetzbar und wird in der vorliegenden Erfindung in modularen Systemen integriert, die verschiedene Anwendungen ermöglichen. Ein solches UV-C-Modul bzw. ein modulares System, umfassend ein oder mehrere solcher UV-C-Module können somit unter anderem zur Desinfektion bzw. Entkeimung von Luft in Autos, Bussen, Bahnen, Gondeln, Restaurants, Hotels, Schulgebäuden, Büros, Hallen oder sonstigen räumlich abgeschlossenen Bereichen verwendet werden.

Ein UV-C-Modul der Erfindung umfasst mindestens einen linearen Strömungskanal, sowie mindestens einen UV-C-Strahler, der in dem Strömungskanal angeordnet ist und der die vorbeiströmende Luft mit UV-C-Strahlung behandelt. Die so behandelte Luft ist bei optimaler Strahlungsdosis und -dauer frei von aktiven Krankheitserregern. Das UV-C-Modul umfasst weiterhin mindestens eine erste Lichtfalle, einen Ventilator, ein Filterflies und eine zweite Lichtfalle.

Das UV-C-Modul der vorliegenden Erfindung ist eckig gestaltet, d.h. es liegt als Würfel oder Quader vor.

In Ausgestaltung der Erfindung kann weiterhin vorgesehen sein, dass die Innenwände des Strömungskanals im Bereich der Bestrahlungsstrecke, d.h. dort wo die durchströmende Luft durch die vom UV-C-Strahler abgegebene Strahlung behandelt wird, zumindest bereichsweise eine die UV-C-Strahlung reflektierende Oberfläche aufweisen. Durch die Wandreflektion der UV-C-Strahlung können die UV-C-Dosis und damit der Wirkungsgrad der Keimtötung wesentlich er höht werden. Ein solches UV-C-Strahlung reflektierendes Material ist beispielsweise Aluminium, das die UV-C- Strahlung zu mindestens 40% reflektiert.

In Ausbildung der Erfindung ist vorgesehen, dass die UV-C-Strahler als nicht ozonbildende Quecksilber-Niederdruckentladungsstrahler mit einer Primärstrahlung von 253,7 nm ausgebildet sind. Diese Art von Strahlern hat gegenüber ozonbildenden Strahlern den Vorteil, dass kein Ozon innerhalb und außerhalb des UV-C-Moduls auftritt. Alternativ können, etwa zur Luftreinigung in Außenbereichen (Geruchsneutralisation) auch Ozon generierende Strahler eingesetzt werden. Neben Niederdruckstrahlern besteht ebenfalls die Möglichkeit Mitteldruckstrahler einzusetzen. Weiterhin kann das Modul auch mit Hochleistungs-LED's zur Erzeugung der germiciden /katalytischen Induktionsstrahlung ausgestattet werden.

Das Modul ist von einem Gehäuse mit reflektierender Innen-Oberfläche (im Strömungskanal) umgeben, das auf der Außenseite in RAL Farben pulverbeschichtet werden kann. Die Außenseite kann ebenfalls alternativ in gewünschten Farben laminiert werden. Das Gehäuse kann in verschiedenen Längen und Querschnitten gefertigt werden, bis hin zur Miniaturisierung. Die Modulkomponenten sind einfach und variabel in das jeweilige Gehäuse einsetzbar.

In jedem Fall sind Lichtfallen vorhanden, so dass keine UV-C-Strahlung nach außen gelangen kann.

Zusätzlich zur Luftentkeimung können Anteile der UV-C-Strahlung fotokatalytisch genutzt werden. Dies geschieht durch einfaches Aufsetzen von hohlen TiO₂ dotierten Keramikschaumzylindern auf die im Modul installierten UV-C-Strahler. Eine Alternative ist das Auskleiden des Strömungskanals mit TiO₂ dotierten Platten. Hierdurch besteht die Möglichkeit fotokatalytisch Geruchsstoffe aus der Umluft zu entfernen (etwa Fischfeinkost, Wurst und Käsetheken etc.). Ebenfalls besteht die Möglichkeit in Frischebereichen für Obst- und Gemüse das Reifegas "Etylen" fotokatalytisch abzubauen, um die Produkthaltbarkeit zu verlängern.

In Zentralen Anlagen besteht damit weiterhin die Option geruchsaktive Komponenten der Außenluft (z.B. Kerosin, Flughäfen) zu reduzieren.

Modulare Systeme der Erfindung umfassen nun ein, zwei drei, vier, fünf, sechs, sieben, acht, neun oder zehn einzelne UV-C-Module. Bevorzugt sind modulare Systeme die ein, zwei, vier oder sechs UV-C-Module umfassen. Sie werden auch als Ein-Modul-System, Zwei-Modul-System, Vier-Modul-System und Sechs-Modul-System bezeichnet.

Ein bestehendes modulares System kann jederzeit durch Hinzufügen oder Wegnehmen eines oder mehrerer UV-C-Module in seiner Größe und Leistungsfähigkeit verändert und den jeweiligen Bedürfnissen angepasst werden. Das Gesamtsystem kann dann mit einer formschönen Frontplatte abgedeckt werden.

Die einzelnen Module weisen ein Kopplungssystem auf.

Die Verbindung mehrerer Module miteinander erfolgt dabei über ein elektronisches Kopplungssystem. Das elektronische Kopplungssystem umfasst elektrische Steckerverbindungen.

Die Kopplung der Module erweist sich als entscheidender Vorteil gegenüber anderen Systemen. Diese Kopplung ermöglicht die Modularität des erfindungsgemäßen Systems, d.h. die einfache Kombinierbarkeit mehrerer Module zu einem größeren Gesamtsystem, um beispielsweise eine Anpassung an Raumgrößere vorzunehmen oder wenn eine größere Personengruppe in einem Raum zusammenkommt.

Die quadratisch / rechteckigen Gehäusegeometrien ermöglichen zusammen mit der Kopplungsmöglichkeit der Module, mehrere Module in einfacher Art und Weise zu einem Gesamtsystem aus mehreren Systemeinheiten zu verbinden.

Die einzelnen Module werden vorzugsweise durch eine elektrische Steckerverbindung miteinander gekoppelt, wobei die Module eine Stromversorgung haben.

Die Steuerung der Module erfolgt entweder einzeln oder aber zentral durch eines der Module.

Ein UV-C-Modul kann in einer Ausführungsform auf dem Gehäuse eine Empfängersteckdose, wie auch einen elektrischen Ausgangs-Stecker besitzen.

Bisher verfügbare Geräte weisen unvorteilhafterweise häufig einen durchgängig sehr hohen Geräusch- und Lärmpegel auf. Dieser ist vor allem beim Betrieb der Geräte an "geräuschempfindlichen" Orten, wie etwa Schulen, Hotels, Gaststätten, Praxisräumen, oder Büros, problematisch. Deshalb können UV-C-Module oder modulare Systeme der Erfindung von einem Schallschutzgehäuse umgeben sein oder einen elektronischen Schallwandler umfassen (Interferenzprinzip). Ein elektronischer Schallwandler arbeitet dabei mit dem sogenannten Gegenschall. Es werden um 180 Grad zum ursprünglichen Geräusch phasenverschobene Schallwellen erzeugt und somit Geräusche deutlich, d.h. um bis zu 95%, reduziert (ANC-Verfahren / Active Noise Control).

Zusätzlich kann eine geräuschoptimierte Variante des UV-C-Moduls einen geräuschoptimierten Ventilator umfassen. In einer bevorzugten Ausführung wird der Ventilator bei 500 U/min betrieben. In einer bevorzugten Ausführung wird der Ventilator bei geringer als der maximalen Ventilatorleistung betrieben. Derartige Ventilatoren weise eine Drehzahlregelung auf und werden bei weniger als 20% oder bei ungefähr 20 - 35 %, vorzugsweise genau 20 % der maximalen Ventilatorleistung (=der maximalen Drehzahl) betrieben.

In einer anderen bevorzugten Ausführung wird der Ventilator bei weniger als oder genau 20% der Ventilatorleistung betrieben.

Modulare Systeme der Erfindung können als Standgeräte mit Standfuß bzw. Ständer als Wandelemente durch Wandmontage oder als Deckenelemente durch Deckenaufhängung bereitgestellt werden.

Durch ihre eckige Form und den modularen Aufbau lassen sie sich somit einfach, schnell und flexibel in jeden Raum integrieren und auch wieder entfernen.

Bei geometrisch ungünstigen Räumen können Einzelsysteme verteilt im Raum platziert werden, um optimale Ergebnisse zu erzielen.

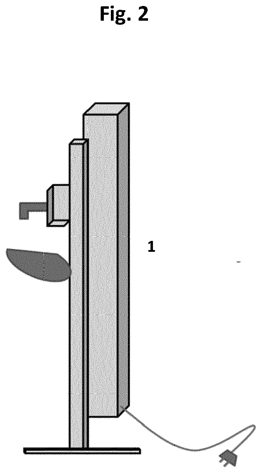

Vorgesehen ist auch ein kombiniertes Desinfektionssystem, das ein UV-C-Modul, also Luftreinigungssystem, mit einem Desinfektionsmittelständer, also Hautdesinfektion, verbindet, so dass eine Desinfektionsinsel entsteht (Fig. 2). Dieses System (eine derartige Desinfektionsinsel) kann in Eingangsbereichen, Anmeldebereich Industrie, Apotheken, Wartezimmern Ärzten, Umkleideräumen, etc. Anwendung finden. Das System ist platzsparend und erfüllt kombinatorisch zwei Funktionen.

Bei Stand und Wandgeräten wird die Luft von dem unten befindlichen Ventilator angesaugt und über eine Filtereinheit durch den Strömungskanal geführt (Fig. 1 B). Die einsetzbaren Filterfliese sind nach ISO Kategorisierung variabel wählbar.

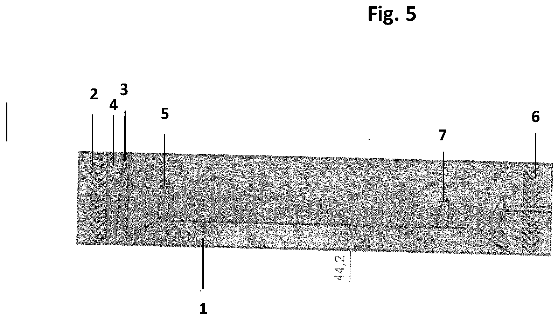

Die Halterungen für die Einzelkomponenten sind auf einem einschiebbaren Montageblech (Fig. 5) auf der Innenseite installiert, so dass die Komponenten einfach eingesteckt werden können. Das Montageblech kann in das Gehäuse des UV-C-Moduls einschiebbar, oder gleichzeitig Gehäusebauteil sein. Der UV-C-Strahler wird zentral fixiert: durch die Sockelfixierung und optional über eine zusätzliche Federklemme. Die Montage erfolgt einfach auf dem Montageblech (Fig. 5), auf dem alle Komponenten angebracht werden.

Zusätzliche elektronische Bauteile können unter dem Blech montiert werden, so dass sie vor der UV-C-Strahlung geschützt sind. Der UV-C-Strahler lässt sich damit einfach wechseln. Das Modul kann am Lufteinlass und Luftauslass durch zusätzliche verschraubbare Gitter vor unsachgemäßem Zugang geschützt werden (Kindersicherung). Als optionales Bauteil kann eine elektronische Steuereinheit integriert werden, die die Möglichkeiten bietet das UV-C-Modul über eine Zeitschaltuhr zu betreiben, stufenlose Einstellungen der Luftvolumenströme vorzunehmen und die Betriebsstunden anzuzeigen. Bei der Kombination mehrerer UV-C-Einheiten werden diese an ein Mastergerät mit Steuerung angeschlossen.

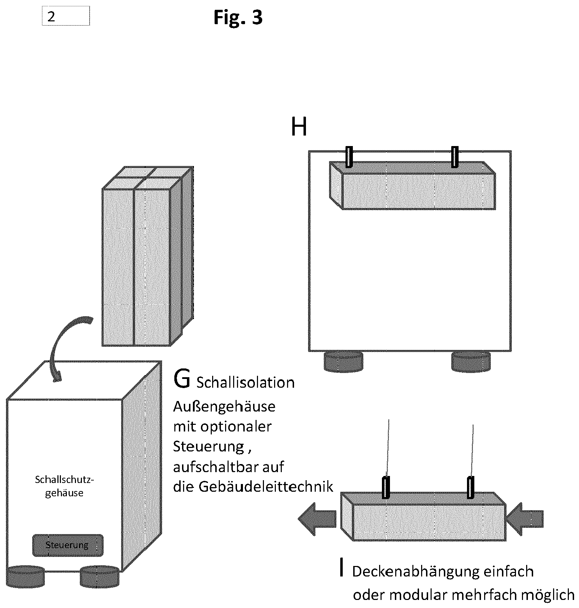

Es besteht weiterhin die Möglichkeit die Systeme mit einer Industriesteuerung auszustatten (Fig. 3G), so dass eine Aufschaltung auf die Gebäudeleittechnik möglich wird. Durch Integration eines CO₂ Sensors in die Steuerung ist optional eine Anpassung der Entkeimungsleistung an die nutzungsabhängige Situation möglich.

Weiterhin können die erfindungsgemäßen UV-C Module auch mit Infrarotschnittstellen, Bluetooth-Schnittstelle oder Wireless Lan (WLAN) - Schnittstellen oder anderen Schnittstellenmodulen ausgerüstet werden.

Die bevorzugten erfindungsgemäßen UV-C Module sind auch durch Sprachsteuerung zu bedienen.

Gemäß einer weiteren Ausführungsform der Erfindung ist vorgesehen, dass die Armaturen der UV-C-Strahler gegen Staub-und Spritzwasser geschützt sind, wodurch das Luftreinigungsgerät allseitig und universell einsetzbar ist (z.B. Arbeitsbereiche mit Spritzwasser (Fleischindustrie)).

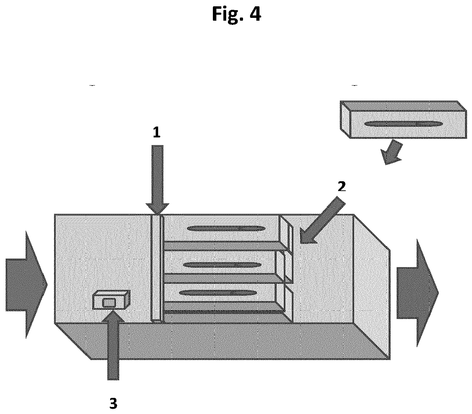

In einer weiteren Ausführungsform können die Einzelmodule ohne Ventilator, Lichtfalle und Filter in bestehende RLT-Zentralanlagen integriert werden, wobei die Quader-Bauform eine Anpassung an alle bestehenden RLT-Anlagen bietet. Durch einfaches kombinieren lässt sich jeder Querschnitt einer zentralen RLT Anlage modular mit den Systemkomponenten ausstatten (Fig. 4).

Alternativ können Einzelmodule oder System der Erfindung, die mehrere Module umfassen, einzelständig sein und an RLT-Anlagen angeschlossen werden.

### Ausführungsbeispiele

Ausführungsbeispiele der hierhin beschriebenen Erfindung werden in Fig. 1 schematisch dargestellt.

Figur 1 zeigt eine schematische Übersicht über verschiedene Ausführungsformen des modularen Systems der Erfindung. A) bis D) stellen sogenannte Ein-UV-C-Modul-Systeme dar. Jeweils ein in rechteckiger Form ausgebildetes UV-C-Modul wird zur Luftdesinfektion bzw. Entkeimung allein verwendet. Ein solches Ein-Modul-System kann z.B. freistehend mit Standfuß (A-C) oder als Wandmontage (D) verwendet werden. Insbesondere wird dargestellt: A) Einzelteile: 1) UV-C-Brenner 1 x 95 Watt oder variabel, 2) Gehäuse quadratisch, innen verspiegelt, 12 cm x 12 cm oder variabel, 3) Filterflies variabel nach ISO, 4) Ventilator, 5) Lichtfalls, 6) Wandhalterung, 7) Standfuß; B) Einzelmodul; C) Standgerät; D) Wandmontage. E) bis F) stellen Systeme dar, die aus mehreren Modulen aufgebaut sind. So ist E) als Drei-Modul-System in Wandmontage aufgebaut, F) als Vier-Modul-System in Wandmontage. Insbesondere wird dargestellt: E) kombiniert in Wandmontage; F) kombiniert in Wandmontage.

Figur 2 zeigt schematisch eine weitere Ausführungsform des modularen Systems der Erfindung. Ein Ein-Modul-System ist dargestellt, das in Kombination mit einer Hautdesinfektionsvorrichtung bereitgestellt werden kann (Desinfektionsinsel). Insbesondere wird dargestellt: Desinfektionsinsel mit 1) UV-C-Modul (Umluftentkeimungsgerät).

Figur 3 zeigt schematisch weitere Ausführungsformen des modularen Systems der Erfindung. G) stellt ein Vier-Modul-System dar, das zusätzlich von einem Schallschutzgehäuse umgeben ist. H) und I) stellen Ein-Modul-Systeme dar, die über eine Aufhängung von oben herab hängend ausgefertigt werden können, z.B. an einer Plexiglasscheibe im Supermarkt (H) oder von der Decke (I). Insbesondere wird dargestellt: G) Schallisolation: 1) Schallschutzgehäuse, 2) Steuerung, 3) Außengehäuse mit optionaler Steuerung, aufschaltbar auf Gebäudeleittechnik; H) Plexiglasschutz, Aufhängung; I) Deckenabhängung einfach oder modular mehrfach möglich.

Figur 4 zeigt eine weitere Ausführungsform des modularen Systems der Erfindung. Hier wird ein Sechs-Modul-System in eine RLT-Anlage integriert. Insbesondere wird dargestellt: 1) Moduladapter, 2) Einzelmodul 6x, 3) Steuerung mit Aufnahme der EVGs.

Figur 5 zeigt schematische eine Ausführungsform des Montageblechs der Erfindung. Auf dem Montageblech (1) sind Steckplätze für Lichtfallen (2) und (6), Ventilator (3), Filterflies (4) und UV-C-Strahler (5), sowie optional eine Klemme zur Fixierung des UV-C-Strahlers (7).

### Offenbarte Aspekte der Erfindung:

1. UV-C-Modul zur Desinfektion bzw. Entkeimung von Raumluft mittels UV-C-Strahlung, umfassend mindestens einen Strömungskanal, eine erste Lichtfalle, einen Ventilator, ein Filterflies, mindestens einen UV-C-Strahler und eine zweite Lichtfalle,
   wobei die den Strömungskanal durchströmende Luft mit UV-C-Strahlung behandelt und die darin enthaltenen Keime inaktiviert werden und weiter umfassend ein Kopplungssystem.
2. Das UV-C-Modul nach Aspekt 1, wobei das UV-C- Modul rechteckig oder quadratisch ausgestaltet ist.
3. Das UV-C-Modul nach einem der vorhergehenden Aspekte, wobei das Modul einen geräuschoptimierten Ventilator umfasst.
4. Das UV-C-Modul nach Aspekt 3, wobei der Ventilator eine Drehzahlregelung umfasst und bei 20 -35 %, vorzugsweise 20 % der maximalen Ventilatorleistung betrieben wird.
5. Modulares System für die Desinfektion bzw. Entkeimung von Raumluft durch Behandlung mit ultravioletter (UV) Strahlung, umfassend mindestens ein UV-C-Modul nach einem der vorhergehenden Ansprüche.
6. Das modulare System nach Aspekt 5, wobei das System zwischen 2 und 8 UV-C-Module umfasst.
7. Das modulare System nach Aspekt 6, wobei das System 2, 3 oder 4 UV-C-Module oder 6 UV-C-Module umfasst.
8. Das modulare System nach einem der vorstehenden Aspekte, wobei das Kopplungssystem ein elektrisches Kopplungssystem umfasst.
9. Das modulare System nach einem der Aspekte 5-8, wobei das System ferner einen Standfuß oder Ständer umfasst und als Standgerät ausgestaltet ist.
10. Das modulare System nach einem der Aspekte 5-8, wobei das System ferner eine Wandaufhängung umfasst zur Wandmontage ausgestaltet ist oder eine Deckenaufhängung umfasst und zur Deckenaufhängung ausgestaltet ist.
11. Desinfektionssystem bestehend aus einem modulares System nach einem der Aspekte 5-10, vorzugsweise mit 1, 2, 3 oder 4 UV-C-Module und weiter umfassend einen Standfuß oder Ständer, vorzugsweise aus Edelstahl und weiter umfassend ein Desinfektionsmodul zur Hautdesinfektion, welches ebenfalls am dem Standfuß befestigt wird.
12. Das modulare System nach einem der Aspekte 5-10 oder das Desinfektionssystem nach Anspruch 11, wobei das System ferner einen elektrischen Schallwandler oder ein Schallschutzgehäuse umfasst.
13. Das modulare System nach einem der Aspekte 5-10, wobei das System in eine RLT-Anlage eingebaut ist oder an diese angeschlossen ist.
14. Das modulare System nach einem der Aspekte 5-10, wobei die UV-C-Module mit Infrarotschnittstellen, Bluetooth-Schnittstelle oder Wireless Lan (WLAN) -Schnittstellen ausgestattet sind.
15. Montageblech für den Aufbau eines UV-C-Moduls, umfassend Steckplätze für eines oder mehrere von mindestens ein Gitter, eine Lichtfalle, einen Ventilator, ein Filterflies und einen UV-C-Strahler.
16. Das Montageblech nach Aspekt 15, wobei die Steckplätze in der folgenden Reihenfolge angeordnet sind:
   1) Gitter
   2) Lichtfalle
   3) Ventilator
   4) Filterflies
   5) UV-C-Strahler
   6) Lichtfalle
   7) Gitter;
   oder
   1) Gitter
   2) Lichtfalle
   3) Filterflies
   4) Ventilator
   5) UV-C-Strahler
   6) Lichtfalle
   7) Gitter.
17. Bausatz für ein UV-C-Modul, umfassend das Montageblech nach einem der Aspekte 15 und 16 und mindestens ein Gitter, eine Lichtfalle, einen Ventilator, ein Filterflies und einen UV-C-Strahler.
18. Der Bausatz nach Aspekt 17, ferner umfassend ein Schallfiltergehäuse oder einen elektrischen Schallwandler, der mit dem UV-C-Modul verbunden werden kann.
19. Der Bausatz nach Aspekt 17, ferner umfassend einen geräuschoptimierten Ventilator.
20. Der Bausatz nach einem der Aspekte 17 - 19, ferner umfassend mindestens eines der folgenden: eine Wandhalterung, einen Standfuß oder eine Deckenaufhängung.

## Patentansprüche

1. UV-C-Modul zur Desinfektion bzw. Entkeimung von Raumluft mittels UV-C-Strahlung, umfassend mindestens einen Strömungskanal, eine erste Lichtfalle, einen Ventilator, ein Filterflies, mindestens einen UV-C-Strahler und eine zweite Lichtfalle,
wobei die den Strömungskanal durchströmende Luft mit UV-C-Strahlung behandelt und die darin enthaltenen Keime inaktiviert werden und weiter umfassend ein Kopplungssystem, optional wobei das UV-C- Modul rechteckig oder quadratisch ausgestaltet ist.

2. Das UV-C-Modul nach Anspruch 1, wobei das Modul einen geräuschoptimierten Ventilator umfasst, der optional eine Drehzahlregelung umfasst und bei 20 -35 %, vorzugsweise 20 % der maximalen Ventilatorleistung betrieben wird.

3. Modulares System für die Desinfektion bzw. Entkeimung von Raumluft durch Behandlung mit ultravioletter (UV) Strahlung, umfassend mindestens ein UV-C-Modul nach einem der vorhergehenden Ansprüche.

4. Das modulare System nach Anspruch 3, wobei das System zwischen 2 und 8 UV-C-Module umfasst, bevorzugt wobei das System 2, 3 oder 4 UV-C-Module oder 6 UV-C-Module umfasst.

5. Das modulare System nach einem der Ansprüche 3-4, wobei das System ein elektrisches Kopplungssystem umfasst.

6. Das modulare System nach einem der Ansprüche 3-5, wobei das System ferner
a) einen Standfuß oder Ständer umfasst und als Standgerät ausgestaltet ist;
b) eine Wandaufhängung umfasst und zur Wandmontage ausgestaltet ist, oder
c) eine Deckenaufhängung umfasst und zur Deckenaufhängung ausgestaltet ist.

7. Desinfektionssystem bestehend aus einem modulares System nach einem der Ansprüche 3-6, vorzugsweise mit 1, 2, 3 oder 4 UV-C-Modulen und weiter umfassend einen Standfuß oder Ständer, vorzugsweise aus Edelstahl und weiter umfassend ein Desinfektionsmodul zur Hautdesinfektion, welches ebenfalls an dem Standfuß befestigt wird.

8. Das modulare System nach einem der Ansprüche 3-6 oder das Desinfektionssystem nach Anspruch 7, wobei das System ferner einen elektrischen Schallwandler oder ein Schallschutzgehäuse umfasst.

9. Das modulare System nach einem der Ansprüche 3-6, wobei die UV-C-Module mit einer Infrarotschnittstelle, Bluetooth-Schnittstelle oder Wireless Lan (WLAN) - Schnittstelle ausgestattet sind.

10. Montageblech für den Aufbau eines UV-C-Moduls, umfassend Steckplätze für eines oder mehrere von mindestens ein Gitter, eine Lichtfalle, einen Ventilator, ein Filterflies und einen UV-C-Strahler.

11. Das Montageblech nach Anspruch 10, wobei die Steckplätze in der folgenden Reihenfolge angeordnet sind:
1) Gitter
2) Lichtfalle
3) Ventilator
4) Filterflies
5) UV-C-Strahler
6) Lichtfalle
7) Gitter;
oder
1) Gitter
2) Lichtfalle
3) Filterflies
4) Ventilator
5) UV-C-Strahler
6) Lichtfalle
7) Gitter.

12. Bausatz für ein UV-C-Modul, umfassend das Montageblech nach einem der Ansprüche 10 und 11 und mindestens ein Gitter, eine Lichtfalle, einen Ventilator, ein Filterflies und einen UV-C-Strahler.

13. Der Bausatz nach Anspruch 12, ferner umfassend ein Schallschutzgehäuse oder einen elektrischen Schallwandler, der mit dem UV-C-Modul verbunden werden kann.

14. Der Bausatz nach Anspruch 12, ferner umfassend einen geräuschoptimierten Ventilator.

15. Der Bausatz nach einem der Ansprüche 12 -14, ferner umfassend mindestens eines der folgenden: eine Wandhalterung, einen Standfuß oder eine Deckenaufhängung.
